# EUROPEAN PATENT SPECIFICATION

(11) **EP 0 395 441 B1**
(45) Date of publication and mention of the grant of the patent: **15.02.1995**
(21) Application number: 90304638.1
(22) Date of filing: 27.04.1990
(51) Int. Cl.: A61K 31/11, A61K 31/19, A61K 31/215, A61K 31/22

(54) **Treating agent for osteoarthritis**
Behandlungsmittel gegen Osteoarthritis
Agent pour le traitement de l'ostéo-arthrite

(30) Priority: 28.04.1989 JP 110148/89; 15.02.1990 JP 34065/90
(43) Date of publication of application: 31.10.1990
(73) Proprietor: KUREHA KAGAKU KOGYO KABUSHIKI KAISHA, Chuo-ku Tokyo 103 (JP)
(72) Inventor: Kimura, Fumihiko, Tokyo (JP); Mukaida, Yutaka, Iruma-gun, Saitama-ken (JP); Watanabe, Koju, Sakado-shi, Saitama-ken (JP)
(74) Representative: Woods, Geoffrey Corlett

(56) References cited:
- US-A- 4 758 591
- PATENT ABSTRACTS OF JAPAN, vol. 7, no. 175 (C-179)(1320), 3rd August 1983; & JP-A-58 83 619 (KUREHA KAGAKU KOGYO K.K.) 19-05-1983
- "THE MERCK MANUAL, 15th edition, pages 1239-1267;
- JOURNAL OF CLINICAL IMMUNOLOGY, vol. 5, no. 5, 1985, pages 287-297, Plenum Publishing Corporation; C.A. DINARELLO: "An update on human interleukin-1: from molecular biology to clinical relevance"

## Description

The present invention relates to the use of a compound represented by the formula (I): 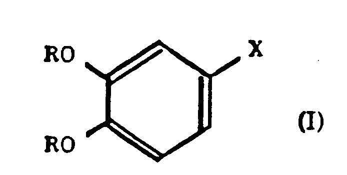
wherein R represents a hydrogen atom or an acyl group; X represents a CHO group, a COOH group or a physiologically acceptable salt thereof, or a CH(OR')₂ group wherein R' represents an acyl group, in the manufacture of a medicament for use in the treatment of osteoarthritis or for use in the suppression of release of proteoglycan in the cartilage matrix.

As an arthrosis, rheumatoid arthritis, rheumatic fever and osteoarthritis are common. Of these three disorders, rheumatoid arthritis and rheumatic fever have been the more extensively studied. However, recently as there has been an increase in the proportion of aged people in the total population, patients suffering from a primary osteoarthritis due to a senescence of a joint, especially to a deformation of cartilage by detrition are increasing. Accordingly the disease is a subject of interest to many physicians.

Up to now, among the compounds represented by the formula (I) 3,4-dialkanoyloxybenzylidene dialkanoate including 3,4-diacetoxybenzylidene diacetate (hereinafter referred to as "ACP"), 3,4-dialkanoyloxybenzaldehyde including 3,4-dihydroxybenzaldehyde (hereinafter referred to as "PAL") and 3,4-dialkanoyloxybenzoic acid including 3,4-dihydroxybenzoic acid (hereinafter referred to as "PAC") have been described as effective anti-inflammatory agents, treating agents for nephritis, anti-cancer agents and agents for suppressing fibrogenesis. For example, US-A-4,758,591 discloses ACP as an anti-inflammatory agent, JA-A-55-51,018 (1980) discloses PAL as an anti-cancer agent, JP-A-58-83,619 (1983) discloses PAL as an anti-inflammatory agent, JP-A-59-196,818 (1984) discloses PAL as an anti-nephritis agent and US-A-4,248,892 discloses PAC as an anti-fibrogenesis agent.

US-A-4,758,591 indicates that 3,4-dialkanoyloxybenzylidene dialkanoates, including ACP and PAL, are appropriate agents for treating inflammatory, rheumatic disease such as rheumatoid arthritis; and autoimmune diseases such as glomerular nephritis or systemic lupus erythematosus, since they suppress activity of leucocyte migration, granuloma proliferation and adjuvant arthritis and have very low toxicity to mammals including humans. However, the above references do not say anything at all about osteoarthritis.

Osteoarthritis is a retroplasia which occurs in a joint. There are secondary osteoarthritis due to a congenital hypoplasia, trauma or other diseases and primary osteoarthritis due to a retroplasia of joint cartilage. In general, osteoarthritis occurs mostly at a joint more or less supporting body weight. Secondary and congenital osteoarthritis are found frequently at a hip joint and primary osteoarthritis due to senescences is found often at a spine joint or a knee.

Osteoarthritis is sometimes classified as a rheumatic diseases in its broad meaning, but it is a different disease from so-called rheumatism such as rheumatoid arthritis and rheumatic fever. For example, rheumatic fever is accompanied with a lesion in the heart and rheumatoid arthritis has an adhesion in the joint. However, osteoarthritis causes a deformation of cartilage of a joint but no adhesion in the joint nor a lesion in the heart. Furthermore, rheumatoid arthritis is not limited to a joint having a load such as a knee joint or a hip joint and its pathopoiesis is often observed at a hand or finger joint. Accordingly, osteoarthritis is different from rheumatism and should be classified separately from rheumatism.

Furthermore, rheumatoid arthritis is a systemic disease and causes an inflammation in connective tissues. Administration of a conventional anti-inflammatory agent is one of the main treatments. Osteoarthritis also causes an inflammation in a synovial membrane but it is not a main lesion and accordingly simple administration of conventional anti-inflammatory agents does not give sufficient results for an adequate treatment.

The present inventors have studied extensively to develop a pharmaceutical drug to treat osteoarthritis, which disease is gaining the attention of many physicians since aging phenomena have recently become remarkable throughout advanced countries. Taking into their consideration a recent finding that interleukin 1 (hereinafter referred to as "IL-1") is noted as a joint cartilage destroying factor, they have created a concept that they should develop a drug which is capable of suppressing the production or release of IL-1 by macrophages.

Based on this concept, the inventors have found that 3,4-dialkanoyloxybenzylidene dialkanoates including ACP, PAL, PAC and its physiologically acceptable salt strongly suppress the production and/or release of IL-1. They have also found that the above compounds can suppress the decomposition of cartilage matrix and the release of proteoglycan, which is a main constituent of the cartilage, as its fragment.

In the accompanying drawings:
Figures 1(a) and 1(b) show the experimental results of inhibiting activities of ACP, PAL and PAC, on IL-1 production in human synovial cells. The values are shown in the form of a mean value ± standard error (n=3) on bar graphs.
Figures 2(a) and 2(b) show the experimental results of the inhibiting effects of ACP,
   3,4-di-n-propionyloxybenzylidene di-n-propionate,
   3,4-di-n-dodecanoyloxybenzylidene di-n-dodecanoate,
   3,4-di-n-octadecanoyloxybenzylidene di-n-octadecanoate,
   3,4-dibenzoyloxybenzylidene dibenzoate, PAL and PAC on IL-1 production in human monocytes. The values are shown in the form of a mean value ± standard error (n=3) on bar graphs.

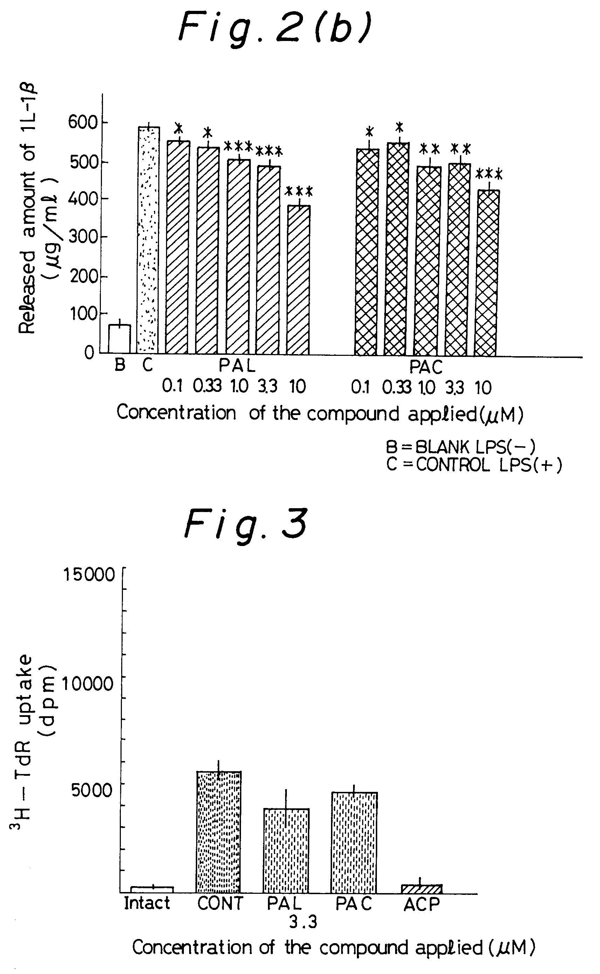

Figure 3 shows the experimental results of the inhibiting effects of ACP, PAL and PAC on IL-1 release from cultured human cartilage cells. The values are shown in the form of a mean value ± standard error n=3) on bar graphs.

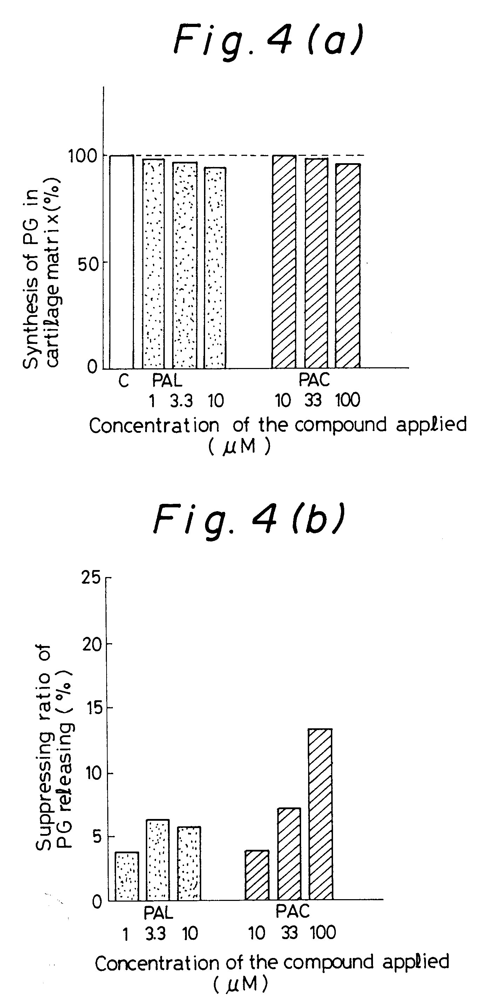

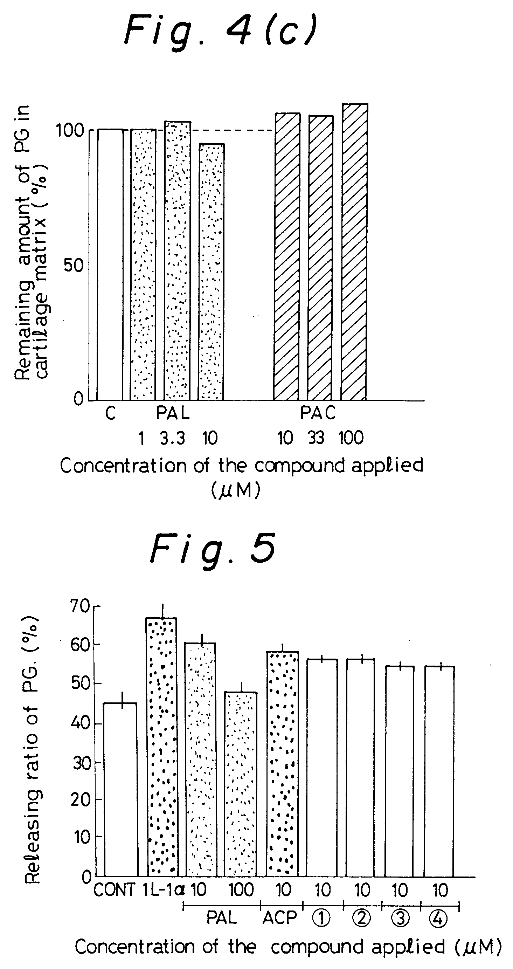

Figures 4(a) and 4(b) show the experimental results of the inhibiting effects of PAL and PAC on synthesis, release and remaining ratios of proteoglycan in or from cartilage matrix.
Figure 5 shows the experimental results of the inhibiting effects of PAL, ACP,
   3,4-di-n-propionyloxybenzylidene di-n-propionate,
   3,4-di-n-dodecanoyloxybenzylidene di-n-dodecanoate,
   3,4-di-n-octadecanoyloxybenzylidene di-n-octadecanoate and
   3,4-dibenzoyloxybenzylidene dibenzoate, on proteoglycan release under the existence of IL-1a. The values are shown in the form of a mean value ± standard deviation (n=3) on bar graph.

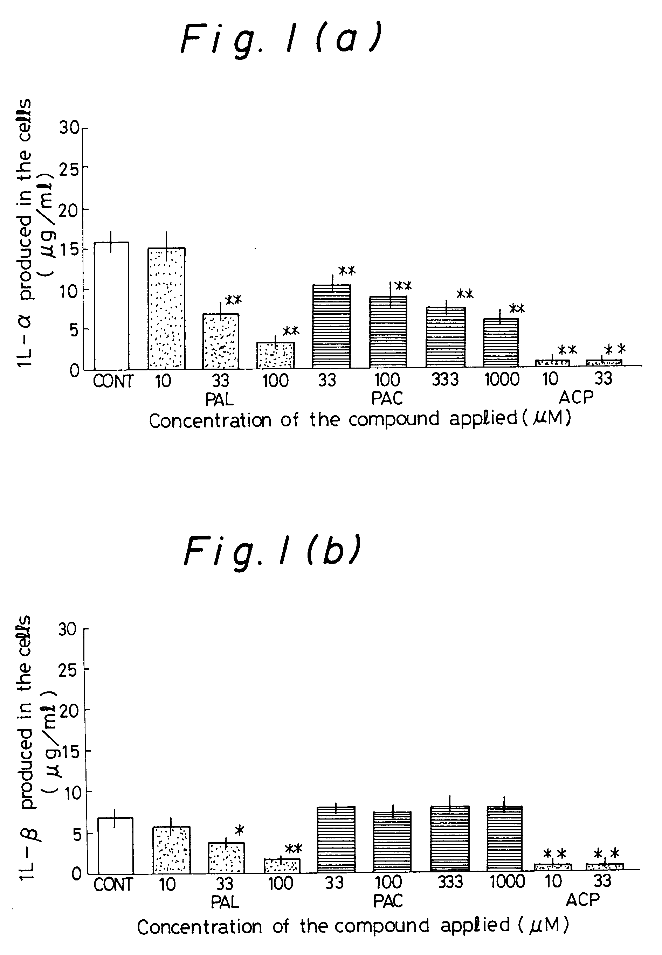

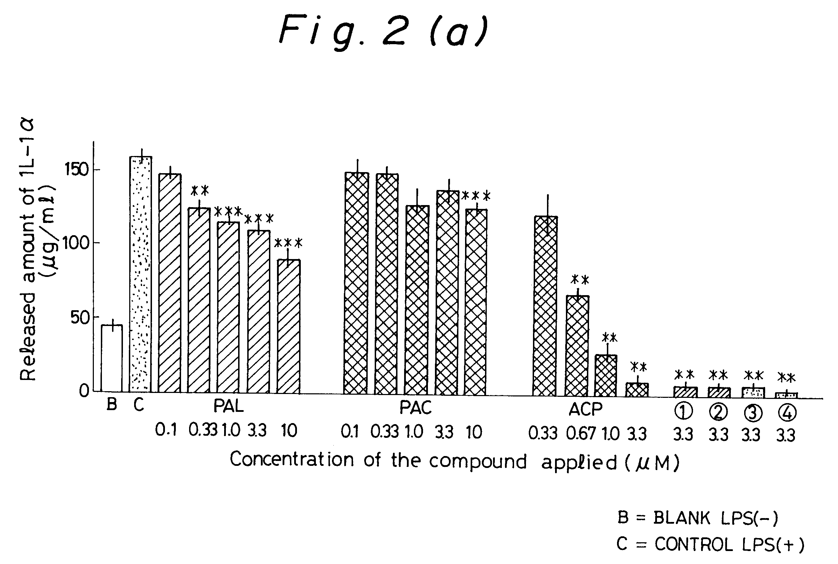

In Figures 1 and 2, each symbol *, ** and *** shows a difference with a level of significance of not higher than 5%, not higher than 1% and not higher than 0.1%, respectively.

The present invention provides the use of at least one compound represented by the formula (I): 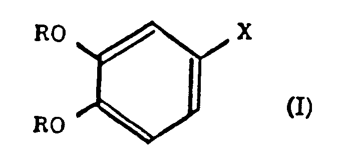
wherein R represents a hydrogen atom or an acyl group of formula: 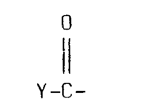
wherein Y represents a straight chain or branched alkyl group having from 1 to 18 carbon atoms or an aromatic residue; X represents a CHO group, a COOH group, or a CH(OR')₂ group wherein R' represents an acyl group of formula: 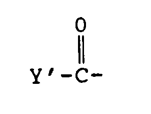
wherein Y' represents a straight chain or branched alkyl group having from 1 to 18 carbon atoms or an aromatic residue; or, when X represents a COOH group, a physiologically acceptable salt thereof, in the manufacture of a medicament for use in the treatment of osteoarthritis.

The present invention also provides the use of a compound of formula (I) or a physiologically acceptable salt thereof in the manufacture of a medicament for use in the suppression of release of proteoglycan in the cartilage matrix.

In formula (I), R and R' acyl groups respectively represent 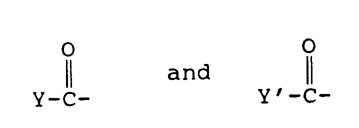
wherein Y and Y' independently represent a straight chain or branched alkyl group having from 1 to 18, preferably from 1 to 7, more preferably 1 or 2, carbon atoms, or an aromatic residue such as a phenyl group or an alkylphenyl group. The alkyl group in the alkylphenyl group may, for example, have from 1 to 7, more preferably 1 or 2, carbon atoms.
The following compounds of formula (I) can be exemplified:
3,4-diacetoxybenzylidene diacetate,
3,4-dipropionyloxybenzylidene dipropionate,
3,4-dibutyryloxybenzylidene dibutyrate,
3,4-didodecanoyloxybenzylidene didodecanoate,
3,4-ditetradecanoyloxybenzylidene ditetradecanoate,
3,4-dihexadecanoyloxybenzylidene dihexadecanoate,
3,4-dioctadecanoyloxybenzylidene dioctadecanoate,
3,4-diacetoxybenzylidene dioctadecanoate,
3,4-dioctadecanoyloxybenzylidene diacetate,
3,4-dibenzoyloxybenzylidene dibenzoate,
3,4-dihydroxybenzaldehyde,
3,4-diacetoxybenzaldehdye,
3,4-dioctadecanoyloxybenzaldehyde,
3,4-dibenzoyloxybenzaldehdye,
3,4-dihydroxybenzoic acid and its physiologically acceptable salts, and
3,4-diacetoxybenzoic acid and its physiologically acceptable salts.
3,4-Dialkanoyloxybenzylidene dialkanoate, including ACP, is a compound represented by the formula (II): 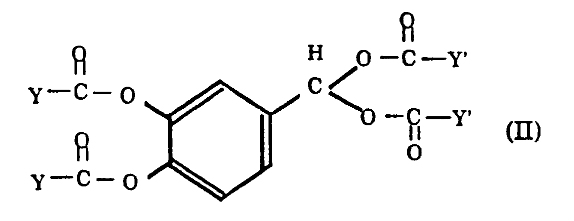
Its method of synthesis is described in US-A-4,758,591 and is summarized below:
(1) When Y and Y' are identical (Y = Y'), for example, PAL represented by the formula (III) is reacted with an alkanoic acid anhydride represented by the formula (IV), in the presence of a mineral acid such as sulfuric acid, as illustrated in the following reaction scheme: 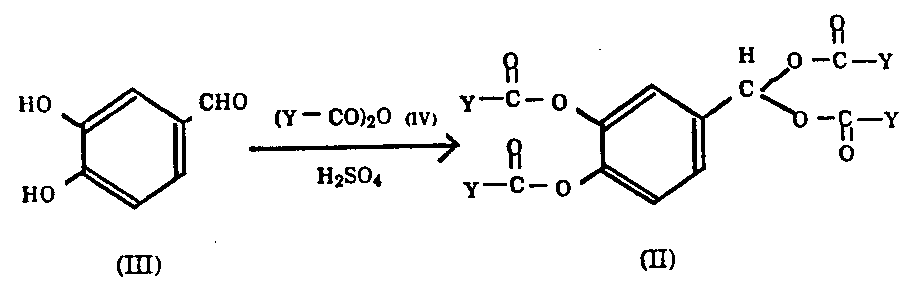
(2) when Y and Y' are different, the compound, for example, represented by the formula (V) is reacted with an alkanoic acid anhydride represented by the formula (VI), in the presence of a mineral acid such as sulfuric acid, as illustrated in the following reaction scheme: 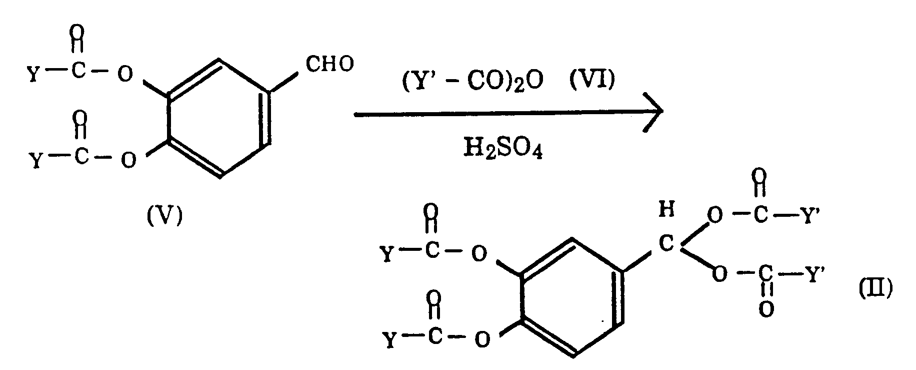
The synthesis of the compound represented by the formula (V) is performed, for example, by reacting PAL represented by the formula (III) with an alkanoic acid halide of the formula (VII), Y-COZ, wherein Z represents a chlorine atom or a bromine atom, in the presence of, for example, triethylamine or pyridine in an inactive organic solvent such as benzene or dichloromethane, as illustrated in the following reaction scheme: 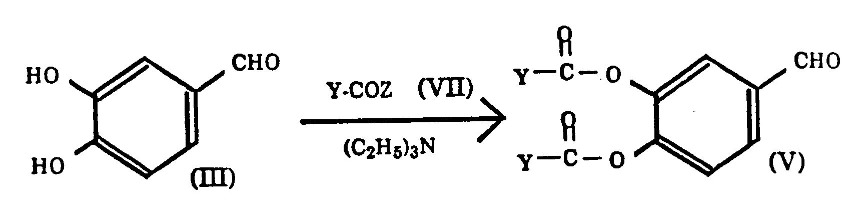
PAC is a compound represented by the formula (VIII): 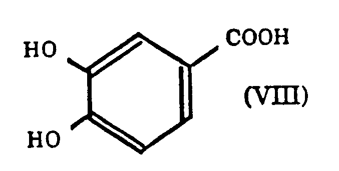
and as its physiologically acceptable salts, those with alkali metals such as the sodium salt and the potassium salt, and those with alkaline earth metals such as calcium the salt and the ammonium salt can be exemplified.

3,4-Dialkanoyloxybenzoic acid represented by the formula (IX) can be prepared by reacting PAC represented by the formula (VIII) with an alkanoic acid anhydride of the formula (IV) in the presence of a mineral acid such as sulfuric acid, as illustrated in the following reaction scheme: 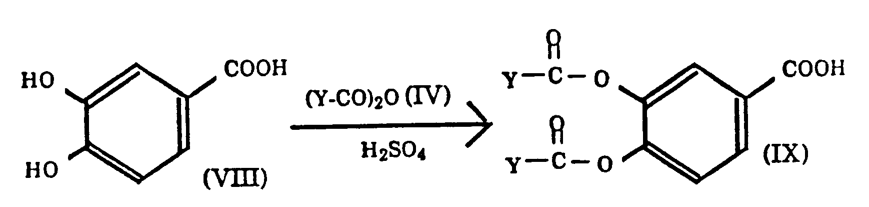
The toxicological and pharmacological features of the compounds of formula (I) are now further explained.

### 1. Acute Toxicity

### (i) Acute toxicity of ACP:

ACP was administered orally or subcutaneously to mice and rats, male and female, their clinical signs were observed for seven days after the administration and each LD₅₀ was determined by the method calculating on the Litchfield-Wilcoxon figure.

For oral administration, a suspension of ACP dispersed in an aqueous solution of 0.5% methylcellulose and 0.4% Tween 80 (nonionic surfactant, manufactured by TOKYO KASEI, Co., Ltd.) was prepared and administered to the animals via a stomach sonde. For subcutaneous administration, a suspension of ACP dispersed in an aqueous physiological saline solution containing 0.5% of carboxymethyl cellulose sodium (hereinafter referred to as "CMC") was prepared and injected to the animals with a syringe. The results are shown in Table 1.

**Table 1**

| Route* | Oral | | | | Subcutaneous | | | |
|---|---|---|---|---|---|---|---|---|
| Animal Tested | CD-1/Mouse | | Sprague-Dawley/Rat | | Jcl:ICR/Mouse | | Jcl:Wistar/Rat | |
| Sex | male | fem.* | male | fem.* | male | fem.* | male | fem.* |
| No. of Animals | 5 | 5 | 5 | 5 | 10 | 10 | 10 | 10 |
| LD₅₀ (g/kg) | >5 | >5 | >5 | >5 | >4 | >4 | >4 | >4 |

| | | | | | | | | |
|---|---|---|---|---|---|---|---|---|
| fem.*: means female | | | | | | | | |

The acute toxicities (LD₅₀) of
3,4-di-n-propionyloxybenzylidene di-n-propionate,
3,4-di-n-dodecanoyloxybenzylidene di-n-dodecanoate,
3,4-di-n-octadecanoyloxybenzylidene di-n-octadecanoate,
3,4-dibenzoyloxybenzylidene dibenzoate,
3,4-di-n-butyryloxybenzylidene di-n-butyrate,
3,4-di-n-tetradecanoyloxybenzylidene di-n-tetradecanoate,
3,4-di-n-hexadecanoyloxybenzylidene di-n-hexadecanoate,
3,4-diactoxybenzylidene di-n-octadecanoate,
3,4-di-n-octadecanoyloxybenzyliden diacetate,
3,4-diacetoxybenzaldehyde,
3,4-di-n-octadecanoyloxybenzaldehyde,
3,4-dibenzoyloxybenzaldehyde and
3,4-diacetoxybenzoic acid and its sodium salt were also determined in the same manner and approximately the same values were obtained.

### (ii) Acute toxicity of PAL:

PAL was administered orally or intraperitoneally to each of five female Jc1:ICR mice per group, their situation was observed for seven days after the administration and each LD₅₀ was determined by the method calculating on the Litchfield-Wilcoxon figure.

For oral administration, a suspension of PAL dispersed in an aqueous solution of 0.2% CMC was prepared (part of the PAL was dissolved in the solution) and administered to the animals via a stomach sonde. For intraperitoneal administration, a suspension of PAL dispersed in an aqueous physiological saline solution was prepared (part of the PAL was dissolved in the solution) and injected in the animals with a syringe. The results are shown in Table 2.

**Table 2**

| Route for Administration | LD₅₀ Value (mg/kg) |
|---|---|
| Oral | 1,503 |
| intraperitoneal | 404 |

### (iii) Acute Toxicity of PAC:

PAC was administered interperitoneally to each of five male mice per group, their clinical signs were observed for 8 days after the administration and an LD₅₀ value of not less than 400 mg/kg was obtained by the same method as in (ii) above. The same level of LD₅₀ was also obtained for a sodium salt of PAC.

### (2) Inhibition of a Production and Release of IL-1:

### (i) Inhibiting activity of ACP, PAL and PAC on IL-1 production in human synovial cells.

Human synovial membrane obtained in an operation was treated with collagenase to obtain synovial cells, which were subcultured several times and inoculated on a 12-well culture dish. On reaching confluence, the cells were supplied to the test described below. The synovial cells produce IL-1 but release only a little amount and accordingly are appropriate cells to study the inhibition or acceleration of IL-1 intracellular production.

DMSO (dimethylsulfoxide) solutions containing one of ACP, PAL and PAC separately in a concentration of 400 times that of the final test and a DMSO solution not containing any of the compounds as a control, were each diluted to 40 times in its volume with a medium containing fetal bovine serum (hereinafter referred to as "FBS") [Ham Medium]. 100»l of the diluted solution was added to each well and 800 »l of the medium [Ham] containing tetradecanoylphorbol acetate (hereinafter referred to as "TPS") and Ca²⁺ ionophore A 23187 was added to the each well. The concentrations of TPA and the ionophore in the medium were adjusted so that their final concentrations in the well became 0.1 »g/ml and 0.2 »g.ml respectively, and production of IL-1 was stimulated. The final volume of the liquid (medium) per well was 1 ml/well. After 40 hours of culture, the liquid portion was removed from each well and 1 ml of a Ham medium was added to the remaining cells in each well. Freeze-thawing was repeated on the medium containing the cells several times to destroy all the cells in the medium and supernatants containing intracellular substances were obtained by centrifugation. IL-1α and IL-1β in the supernatant were determined by the ELISA method using a prescribed enzyme-antibody-complex (manufactured by OHTSUKA SEIYAKU, Co., Ltd.) as intracellular IL-1s [refer to ENSHO, vol. 8 (5) 409 (1988)]. The results are shown in Figures 1(a) and 1(b). As is clear from the Figures, 10»M of ACP and 33»M of PAL show a strong inhibiting activity of both IL-1α and IL-1β production, and 33 »M of PAC shows an inhibiting activity of IL-1α only.

### (ii) Inhibiting activity of the compound formula (I) on a release of IL-1 from human monocyte

Monocytes were isolated from human venous blood by density-gradient centrifugation using Ficoll-paque solution and cells having a purity of not less than 95% were obtained by purifying with a monocyte-separating plate. After dispersing the cells in a medium, RPMI 1640, containing 10% of FBS, the monocytes were inoculated in a culture dish in an amount of 10⁶ cells/3 ml/well and cultured under an atmosphere containing 5% CO₂ at 37°C for 30 minutes. Then, 15 »l of DMSO solutions of one of ACP, PAL and PAC were added to each well (a final DMSO concentration of 0.5%) and 30 minutes later a lipopolysaccharide (hereinafter referred to as "LPS"), a substance to stimulate IL-1 production, was added at a final LPS concentration of 10 »g/ml. The cells were cultured for 42 hours. Then, the reacted culture medium was recovered and accompanied cells with the medium were separated by centrifugation and a supernatant free of cells was obtained. IL-1α and IL-1β released from the cells were measured by the ELISA method in the same manner as in (i) above. As are shown in Figures 2(a) and 2(b), ACP, PAL and PAC significantly inhibit the release of IL-1 at concentrations of 0.67, 0.33 and 10 »M, respectively.

As is shown in Figure 2(a) at the right end, 3,4-di-n-propionyloxy-benzylidene di-n-propionate [indicated as 1], 3,4-di-n-dodecanoyloxybenzylidene di-n-dodecanoate [indicated as 2], 3,4-di-n-octadecanoyloxybenzylidene di-n-octadecanoate [indicated as 3] and 3,4-dibenzoyloxybenzylidene di-benzoate [indicated as 4] also significantly inhibit IL-1 release at a concentration of 3.3 »M.

The following compounds,
3,4-di-n-butyryloxy-benzylidene di-n-butyrate,
3,4-di-n-tetradecanoyloxy-benzylidene di-n-tetradecanoate,
3,4-di-n-hexadecanoyloxy-benzylidene di-n-hexadecanoate,
3,4-diacetoxybenzylidene di-n-octadecanoate,
3,4-di-n-octadecanoyloxy-benzylidene diacetate,
3,4-diacetoxybenzaldehyde,
3,4-di-n-octadecanoylxybenzaldehyde,
3,4-dibenzoyloxybenzaldehyde,
3,4-diacetoxybenzoic acid and its sodium salt, and
sodium salt of PAC
were also observed to have a similar activity.

### (iii) Inhibiting activity on IL-1 release from cultured cartilage cells:

Cartilages were aseptically taken out from the knee and shoulder joints of a young rabbit, treated with a Ham medium containing 0.2% collagenase and separated into single cells. After washing two times with a Ham medium containing 10% FBS, the cells were dispersed into the Ham medium containing 10% FBS at a concentration of 10⁵ cells/ml. DMSO solutions containing one of PAL, PAC and ACP were added to the wells to a final concentration of each compound of 3.3 »M (corresponding to a final DMSO concentration of 0.25%) and then 0.1 »g/ml of TPA and 0.2 »g/ml of Ca²⁺ ionophore A 23187 were added to stimulate the release of IL-1 from the cartilage cells. After 24 hours of cultivation, IL-1 released into each of the supernatants was measured by a bioassay applying an incorporated amount of ³H-thymidine (³H-TdR) as an index. As are shown in Figure 3, PAL, PAC and ACP inhibit the release of IL-1 from the cartilage cells. In the Figure, "Intact" means that TPA and the ionophore were not added and accordingly there was no stimulation of IL-1 release.

### (3) Inhibition on release of proteoglycan (hereinafter referred to as "PG") from cultured cartilage cells:

Costal cartilage was aseptically taken out from 4 weeks old male SD rats, separated by enzymatic treatment into single cells, and then inoculated into a 12-well culture dish and cultured under an atmosphere containing 5% CO₂ at 37°C. After the chondrocytes had been grown to confluence, 1 to 10 »M of PAL or 10 to 100 »M of PAC was added and three hours later, 2 »Ci of Na₂³⁵SO₄ was further added to each well. Twenty four hours later, the synthesis amount in, release amount from and remaining amount of PG in the matrix were determined using the amount of ³⁵S as an index. As are shown in Figures 4(a) and 4(b), no effect of each compound on the PG synthesis was observed, and both PAL and PAC exhibited inhibiting activities on the PG release. In the Figures, the PG release in the control was defined as 100%. The amount of PG remaining in the matrix increased in proportion to the amount of PAL and PAC added between the concentrations of 1 and 3.3 »M and 10 and 100 »M, respectively [Figure 4(c)]. ACP showed a similar effect as described above. In Figures 4(a), 4(b) and 4(c), the released amount of PG means the amount representing the PG degraded in and released from cartilage matrixes, which was calculated on a basis of the ³⁵S in the culture medium. The amount of PG remaining means the PG not degraded in the matrixes, and was also calculated in the same way as above. The sum of the released and the remaining amounts of PG is thought to be the amount of synthesized PG. Accordingly, the more amount remaining and the less amount released of PG mean the compound has a greater chondroprotective effect against osteoarthritis.

Furthermore, to a cultured cartilage matrix, in which PG is prelabelled in advance with 1 »Ci of Na₂³⁵SO₄, 20 units of IL-1α was added to each group including a control group and 10 »M of PAL, 100 »M of PAL or 10 »M of ACP was added to each test group, and the effect of each compound on PG release from the cells was studied. As are seen in Figure 5, the addition of IL-1α increased a release of PG by about 20% when compared to a control group (without addition of IL-1) taking the prelabelled amount of the 35S as 100%. Furthermore, PAL inhibited the amount released proportionally to the amount added and ACP also inhibited the amount released.

As can also be seen in Figure 5, 10 »M of each of 3,4-di-n-propionyloxybenzylidene di-n-propionate [indicated as 1], 3,4-di-n-dodecanoyloxybenzylidene di-n-dodecanoate [indicated as 2], 3,4-di-n-octadecanoyloxybenzylidene di-n-octadecanoate [indicated as 3], 3,4-dibenzoyloxybenzylidene dibenzoate [indicated as 4], inhibited the amount of PG released.

The inhibiting activity of each of:
3,4-di-n-butyryloxybenzylidene di-n-butyrate,
3,4-di-n-tetradecanoyloxybenzylidene di-n-tetradecanoate,
3,4-di-n-hexadecanoyloxybenzylidene di-n-hexadecanoate,
3,4-diacetoxybenzylidene di-n-octadecanoate,
3,4-di-n-octadecanoyloxybenzylidene diacetate,
3,4-diacetoxybenzaldehyde,
3,4-di-n-octadecanoyloxybenzaldehyde,
3,4-dibenzoyloxybenzaldehyde,
3,4-diacetoxybenzoic acid and its sodium salt, and
PAC and its sodium salt,
were also studied and gave the same effects.

As described above, the compounds of formula (I) and, where appropriate, the physiologically acceptable salts, demonstrated an excellent inhibiting activity against IL-1 production and released together with inhibiting activity against PG release from cartilage cells. Thus compounds show a protective action for joint cartilage and low toxicity (side effect). Accordingly the compounds are extremely useful as therapeutic agents for osteoarthritis, including osteogonarthritis, osteoanconitis, malum coxae deformans and spondylosis deformans.

The above compounds can be used in a variety of forms. They can be used singly and also with pharmaceutically acceptable diluents or other agents.

Among the compounds of formula (I), the aldehyde derivatives are often stimulative to a living body and easily oxidized. To overcome these defects, it is useful to make clathrate compounds of the derivative by reacting with, for example, cyclodextrin, or to use the derivatives a mixture with various amines, amino acids or saccharides.

The compounds of formula (I) can be administered orally or parenterally and therefore can take any forms appropriate for such administration. The compounds can also be administered in a wide variety of dosage forms, such as powders, granules, tablets, sugar coated tablets, capsules, suppositories, suspensions, liquids, emulsions, injections and ointments, so long as the forms contain a physiologically effective amount of the compound.

Therefore, a pharmaceutical composition containing the compound of formula (I) as an active ingredient, can be prepared by any publicly known method. The content of the compound of formula (I) in the pharmaceutical composition, as an active ingredient, can be adjusted to from 0.01 to 100%, preferably from 0.1 to 70%, by weight.

The compounds of formula (I) can be administered to humans and animals orally or parenterally. The oral administration includes sublingual and parenteral administration can include subcutaneous, intramuscular, intra-joint cavity, intravenous injections and infusions together with transdermal administration.

The dose of the compound of formula (I) depends on the subject species such as animals and humans, the age, the individual difference, the stage of disorders. Generally speaking, the compound of formula (I) can be administered orally to humans at a dose of from 0.1 to 500 mg/kg body weight/day, preferably from 0.5 to 200 mg/kg body weight/day, and parenterally, it can be administered at a dose of from 0.01 to 200 mg/kg body weight/day, preferably from 0.1 to 100 mg/kg body weight/day. It is preferable to administer the compound from once to 4 times a day.

The preparation of pharmaceutical compositions containing the compound of formula (I) as an active ingredient is further described in the following Examples. The parts given in the Examples are parts by weight, unless otherwise stated.

### Example 1 : Granules

| | |
|---|---|
| ACP | 71 parts |
| Lactose | 17 parts |
| Hydroxypropylcellulose having a low substitution. | 10 parts |
| Hydroxypropylcellulose | 2 parts |

The above components were thoroughly mixed and kneaded using 32 parts of ethanol as an emollient, wet-granulated and dried to obtain the granules.

### Example 2 : Tablets

To the granules prepared in the Example 1, 1% by weight of magnesium stearate was added and mixed well and the mixture was compressed and moulded into the tablets.

### Example 3 : Encapsulated drug

| | |
|---|---|
| PAL | 40 parts |
| Lactose | 50 parts |
| Hydroxypropylcellulose having a low substitution | 10 parts |

The above components were mixed well into a homogenous powder which was packed in capsules to obtain the encapsulated drug.

### Example 4 : Encapsulated drug

The drug was prepared in the same manner as in Example 3, using PAC instead of PAL.

### Example 5 : Injection

| | |
|---|---|
| PAL | 1 part |
| Isotonic sodium chloride solution | 99 parts |

The above components were mixed under heating and sterilized to provide an injectable formulation.

### Example 6 : Injection

An injectable formulation was prepared in the same manner as in Example 5, using PAC instead of PAL.

### Example 7 : Ointment

| | |
|---|---|
| 3,4-Di-n-propionyloxybenzylidene di-n-propionate | 5 parts |
| Purified lanolin | 4 parts |
| White bee wax | 4 parts |
| White vaseline | 87 parts |

The above components were kneaded well to give the ointment.

## Claims

1. Use of at least one compound represented by the formula: 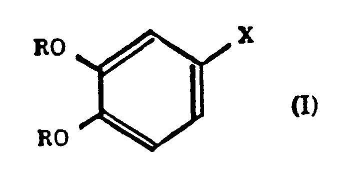 wherein R represents a hydrogen atom or an acyl group of formula: 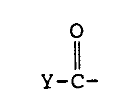 wherein Y represents a straight chain or branched alkyl group having from 1 to 18 carbon atoms or an aromatic residue; and X represents a CHO group, a COOH group, or a CH(OR')₂ group wherein R' represents an acyl group of formula: 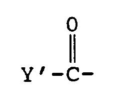 wherein Y represents a straight chain or branched alkyl group having from 1 to 18 carbon atoms or an aromatic residue; or, when X represents a COOH group, a physiologically acceptable salt thereof, in the manufacture of a medicament for use in the treatment of osteoarthritis.

2. Use according to claim 1, wherein said R and R' represent the same acyl group.

3. Use according to claim 1, wherein said R and R' independently represent different acyl groups.

4. Use according to any one of claims 1 to 3 wherein said R and R' respectively represent 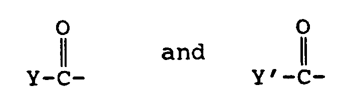 wherein Y and Y' independently represent a branched chain alkyl group having from 1 to 18 carbon atoms.

5. Use according to any one of the preceding claims wherein Y and Y' are independently an alkyl group having from 1 to 7 carbon atoms.

6. Use according to any one of the preceding claims wherein said physiologically acceptable salt is a sodium salt.

7. Use according to any one of the preceding claims, wherein at least one of 3,4-diacetoxybenzylidene diacetate, 3,4-dihydroxybenzaldehyde and 3,4-dihydroxybenzoic acid or a physiologically acceptable salt thereof is used.

8. Use of a compound of formula (I) as defined in any one of the preceding claims or the physiologically acceptable salt thereof in the manufacture of a medicament for use in the suppression of release of proteoglycan in the cartilage matrix.

## Patentansprüche

1. Verwendung mindestens einer Verbindung, dargestellt durch die Formel: 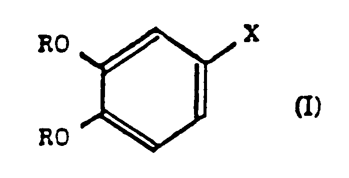 worin R ein Wasserstoffatom oder eine Acylgruppe der Formel: 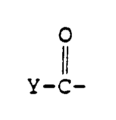 darstellt, worin Y eine geradkettige oder verzweigte Alkylgruppe mit 1 bis 18 Kohlenstoffatomen oder einen aromatischen Rest darstellt; und X eine CHO-Gruppe, eine COOH-Gruppe oder eine CH(OR')₂-Gruppe darstellt, worin R' eine Acylgruppe der Formel: 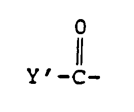 darstellt, worin Y' eine geradkettige oder verzweigte Alkylgruppe mit 1 bis 18 Kohlenstoffatomen oder einen aromatischen Rest darstellt; oder, wenn X eine COOH-Gruppe darstellt, eines physiologisch annehmbaren Salzes davon, bei der Herstellung eines Arzneimittels zur Verwendung bei der Behandlung von Osteoarthritis.

2. Verwendung nach Anspruch 1, worin R und R' die gleiche Acylgruppe darstellen.

3. Verwendung nach Anspruch 1, worin R und R' unabhängig voneinander verschiedene Acylgruppen darstellen.

4. Verwendung nach einem der Ansprüche 1 bis 3, worin die R und R' jeweils darstellen 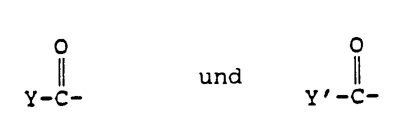 worin Y und Y' unabhängig voneinander eine verzweigtkettige Alkylgruppe mit 1 bis 18 Kohlenstoffatomen darstellen.

5. Verwendung nach einem der vorhergehenden Ansprüche, worin Y und Y' unabhängig voneinander eine Alkylgruppe mit 1 bis 7 Kohlenstoffatomen sind.

6. Verwendung nach einem der vorhergehenden Ansprüche, worin das physiologisch annehmbare Salz ein Natriumsalz ist.

7. Verwendung nach einem der vorhergehenden Ansprüche, worin mindestens eines von 3,4-Diacetoxybenzylidendiacetat, 3,4-Dihydroxybenzaldehyd und 3,4-Dihydroxybenzoesäure oder ein physiologisch annehmbares Salz davon verwendet wird.

8. Verwendung einer Verbindung der Formel (I) wie in einem der vorhergehenden Ansprüche definiert oder des physiologisch annehmbaren Salzes davon bei der Herstellung eines Arzneimittels zur Verwendung bei der Unterdrückung der Freisetzung von Proteoglycan in der Knorpelmatrix.

## Revendications

1. Utilisation d'au moins un composé représenté par la formule : 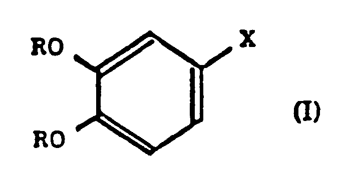 dans laquelle R représente un atome d'hydrogène ou un groupe acyle fe formule : 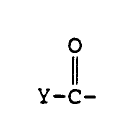 dans laquelle Y représente un groupe alkyle à chaîne droite ou ramifiée, comportant 1 à 18 atomes de carbone, ou un résidu aromatique; et X représente un groupe CHO, un groupe COOH, ou un groupe CH(OR')₂ dans lequel R' représente un groupe acyle de formule : 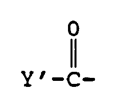 dans laquelle Y représente un groupe alkyle à chaîne droite ou ramifiée comportant 1 à 18 atomes de carbone, ou un résidu aromatique; ou, lorsque X représente un groupe COOH, un sel de ce composé acceptable du point de vue physiologique dans la fabrication d'un médicament pour l'utilisation dans le traitement de l'ostéoarthrite.

2. Utilisation suivant la revendication 1, dans laquelle lesdits groupes R et R' représentent le même groupe acyle.

3. Utilisation suivant la revendication 1, dans laquelle lesdits groupes R et R' représentent des groupes acyle différents.

4. Utilisation suivant l'une quelconque des revendications 1 à 3, dans laquelle lesdits groupes R et R' représentent respectivement les groupes : 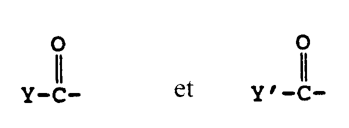 dans lesquels Y et Y' représentent indépendamment une chaîne alkyle ramifiée comportant 1 à 18 atomes de carbone.

5. Utilisation suivant l'une quelconque des revendications précédentes, dans laquelle Y et Y' représentent indépendamment une chaîne alkyle ramifiée comportant 1 à 7 atomes de carbone.

6. Utilisation suivant l'une quelconque des revendications précédentes, dans laquelle ledit sel acceptable du point de vue physiologique est un sel de sodium.

7. Utilisation suivant l'une quelconque des revendications précédentes, dans laquelle au moins un composé parmi le 3,4-diacétoxybenzylidène diacétate, le 3,4-dihydroxybenzaldéhyde et l'acide dihydroxybenzoïque ou un sel de celui-ci, acceptable du point de vue physiologique, est utilisé.

8. Utilisation d'un composé de formule (I) suivant l'une quelconque des revendications précédentes, ou d'un sel de celui-ci, acceptable du point de vue physiologique, dans la fabrication d'un médicament pour l'utilisation dans la suppression de la libération de protéoglycan dans la matrice du cartilage.
